# EUROPEAN PATENT SPECIFICATION

(11) **EP 4 732 934 B1**
(45) Date of publication and mention of the grant of the patent: **09.12.2026**
(21) Application number: 25173441.4
(22) Date of filing: 30.04.2025
(51) Int. Cl.: B01D 53/14, C07D 211/06, C07D 265/00, C07D 295/088, C07D 295/037, C01B 32/50

(54) **CARBON DIOXIDE ABSORBENT, METHOD OF PREPARING THE SAME, AND CARBON DIOXIDE SEPARATION METHOD USING THE SAME**
KOHLENDIOXIDABSORPTIONSMITTEL, VERFAHREN ZU SEINER HERSTELLUNG UND KOHLENDIOXIDABSCHEIDUNGSVERFAHREN DAMIT
ABSORBANT DE DIOXYDE DE CARBONE, SON PROCÉDÉ DE PRÉPARATION ET PROCÉDÉ DE SÉPARATION DE DIOXYDE DE CARBONE L'UTILISANT

(30) Priority: 23.10.2024 KR 20240145516
(43) Date of publication of application: 29.04.2026
(73) Proprietor: SK Innovation Co., Ltd., Seoul 03188 (KR)
(72) Inventor: KIM, Ji Su, 34124 Daejeon (KR); JUNG, Il Gu, 34124 Daejeon (KR); PARK, Eun Joon, 34124 Daejeon (KR); JEONG, Ji Su, 34124 Daejeon (KR); SEO, Sang Hyup, 34124 Daejeon (KR); PYUN, Lim Ok, 34124 Deajeon (KR)
(74) Representative: Prüfer & Partner mbB Patentanwälte · Rechtsanwälte

(56) References cited:
- WO-A1-00/69819
- US-A1- 2002 103 239
- US-A1- 2012 308 457
- US-A1- 2015 118 136

## Description

### TECHNICAL FIELD

The following disclosure relates to a carbon dioxide absorbent containing an ionic material containing a cyclic ammonium cation and a hydroxide anion, a carbon dioxide separation method using the same, a use of the carbon dioxide absorbent in a carbon dioxide separation method, and a method of preparing the carbon dioxide absorbent.

### BACKGROUND

Energy use is increasing worldwide due to the rapid progress in economic development and industrialization, and accordingly, the use of fossil fuels, which are the main source of energy, is also increasing. Global warming issues closely related to energy use are a global concern. Carbon dioxide (CO₂) which accounts for the highest proportion of the major greenhouse gases is mostly produced in a process of burning fossil fuels and converting them into energy.

Since the carbon dioxide produced as such is controllable, a technology to remove carbon dioxide is receiving great attention. A method to be applied to combustion exhaust gas during a process of capturing carbon dioxide is largely divided into an absorption method, an adsorption method, and a membrane separation method according to the separation characteristics. Among them, the most actively used method is an absorption method, and the absorption method is divided into a physical absorption method and a chemical absorption method. In the chemical absorption method, aqueous solutions of amines such as monoethanolamine (MEA), N-methyldiethanolamine (MDEA), and diethanolamine (DEA) have been most widely used, and many studies on synthesis of an ionic material in which a cation and an anion are combined and its use as an absorbent have been conducted.
Therefore, there is an object to develop and provide a carbon dioxide absorbent which exhibits improved properties e.g., regarding carbon dioxide capture (absorption) and economic effectiveness, and also regarding layer separation.
Prior art document US20120308457A1 discloses an alkali carbonate-based carbon dioxide absorbent that contains sterically hindered cyclic amines, as well as a method for removing carbon dioxide comprising: contacting gas containing carbon dioxide with an alkali carbonate-based carbon dioxide absorbent containing an added sterically hindered cyclic amine, absorbing carbon dioxide from the gas, and regenerating the carbon dioxide absorbent.

### SUMMARY

An embodiment of the present disclosure is directed to providing a carbon dioxide absorbent containing an ionic material.

Another embodiment of the present disclosure is directed to providing a carbon dioxide separation method using the carbon dioxide absorbent containing an ionic material.

In one general aspect, a carbon dioxide absorbent contains an ionic material containing a cyclic ammonium cation represented by the following Chemical Formula 1 and a hydroxide anion:

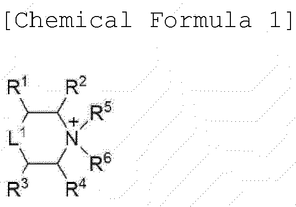

wherein
R¹ to R⁴ are each independently -H, a C₁₋₂₀ alkyl group, a C₁₋₂₀ alkoxy group, a C₁₋₁₀ alkoxy C₁₋₁₀ alkyl group, a C₅₋₂₀ cycloalkyl group, or a 5-membered to 20-membered heterocycloalkyl group; R⁵ and R⁶ are each independently -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group; L¹ is a single bond, -O-, -NR⁷-, or a C₁₋₈ alkylene group; R⁷ is -H or a C₁₋₁₀ alkyl group, or R⁷ may be bonded to R⁵ to form a ring; and the C₁₋₂₀ alkyl group, the C₁₋₂₀ alkoxy group, the C₁₋₁₀ alkoxy C₁₋₁₀ alkyl group, the C₅₋₂₀ cycloalkyl group, and the 5-membered to 20-membered heterocycloalkyl group of R¹ to R⁷ may each independently be substituted with a halogen group, -OH, -NH₂, or -NO₂.

In an exemplary embodiment, in Chemical Formula 1, R¹ to R⁴ are each independently -H, a C₁₋₁₀ alkyl group, a C₁₋₁₀ alkoxy group, a C₁₋₅ alkoxy C₁₋₅ alkyl group, a C₅₋₁₀ cycloalkyl group, or a 5-membered to 10-membered heterocycloalkyl group; R⁵ and R⁶ are each independently -H, a C₁₋₁₀ alkyl group, a C₁₋₅ alkoxy C₁₋₅ alkyl group, a C₅₋₁₀ cycloalkyl group, or a 5-membered to 10-membered heterocycloalkyl group; L¹ is a single bond, -O-, -NR⁷-, or a C₁₋₅ alkylene group; R⁷ is -H or a C₁₋₅ alkyl group, or R⁷ may be bonded to R⁵ to form a ring; and the C₁₋₁₀ alkyl group, the C₁₋₁₀ alkoxy group, the C₁₋₅ alkoxy C₁₋₅ alkyl group, the C₅₋₁₀ cycloalkyl group, and the 5-membered to 10-membered heterocycloalkyl group of R¹ to R⁷ may each independently be substituted with a halogen group, - OH, -NH₂, or -NO₂.

In an exemplary embodiment, the cyclic ammonium cation may be represented by the following Chemical Formula 2:

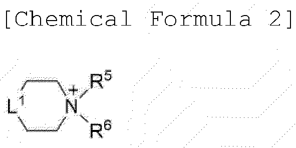

wherein
R⁵ and R⁶ are each independently -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group; L¹ is a single bond, -O-, -NR⁷-, or a C₁₋₈ alkylene group; R⁷ is -H or a C₁₋₁₀ alkyl group, or R⁷ may be bonded to R⁵ to form a ring; and the C₁₋₁₀ alkyl group, the C₁₋₂₀ alkyl group, the C₁₋₁₀ alkoxy C₁₋₁₀ alkyl group, the C₅₋₂₀ cycloalkyl group, and the 5-membered to 20-membered heterocycloalkyl group of R⁵ to R⁷ may each independently be substituted with a halogen group, -OH, -NH₂, or -NO₂.

In an exemplary embodiment, in Chemical Formula 2, R⁵ and R⁶ are each independently a C₁₋₁₀ alkyl group, a C₁₋₅ alkoxy C₁₋₅ alkyl group, a C₅₋₁₀ cycloalkyl group, and a 5-membered to 10-membered heterocycloalkyl group; L¹ is a single bond, -O-, -NR⁷-, or a C₁₋₈ alkylene group; R⁷ is -H or a C₁₋₅ alkyl group; and the C₁₋₁₀ alkyl group, the C₁₋₅ alkoxy C₁₋₅ alkyl group, the C₅₋₁₀ cycloalkyl group, and the 5-membered to 10-membered heterocycloalkyl group of R⁵ to R⁷ may each independently be substituted with -OH, -NH₂, or -NO₂.

In an exemplary embodiment, in Chemical Formula 2, R⁵ and R⁶ are each independently a C₁₋₅ alkyl group or a C₁₋₃ alkoxy C₁₋₃ alkyl group; L¹ is -O- or a methylene group; and the C₁₋₅ alkyl group and the C₁₋₃ alkoxy C₁₋₃ alkyl group of R⁵ and R⁶ may each independently be substituted with -OH.

In an exemplary embodiment, the cyclic ammonium cation may be any one selected from the group consisting of compounds 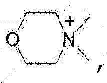 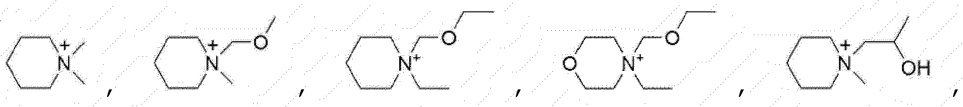 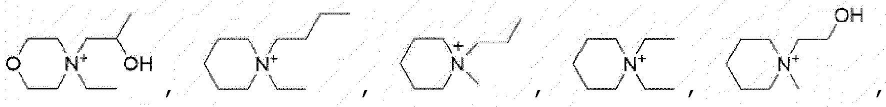 and

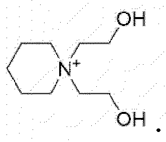

In an exemplary embodiment, the carbon dioxide absorbent may further contain water.

In an exemplary embodiment, the carbon dioxide absorbent may further contain one or more solvents other than the water.

In an exemplary embodiment, the solvent may contain an amine-based compound.

In an exemplary embodiment, the amine-based compound may include one or more selected from the group consisting of monoethanolamine (MEA), N-methyldiethanolamine (MDEA), diethanolamine (DEA), triethanolamine (TEA), 2-amino-2-methyl-1-propanol (AMP), and piperazine (PZ). In a preferred embodiment, the amine-based compound includes piperazine. In a further preferred embodiment, the amine-based compound is piperazine.

In an exemplary embodiment, the ionic material may be contained in the carbon dioxide absorbent in an amount of 5 wt% to 50 wt% with respect to a total weight of the carbon dioxide absorbent. In an exemplary embodiment, the ionic material may be contained in an amount of 20 wt% to 40 wt% with respect to a total weight of the carbon dioxide absorbent. In a preferred embodiment, the ionic material may be contained in an amount of 25 wt% to 30 wt% with respect to a total weight of the carbon dioxide absorbent.

In an exemplary embodiment, the water may be contained in the carbon dioxide absorbent in an amount of 30 wt% to 90 wt% with respect to a total weight of the carbon dioxide absorbent. In an exemplary embodiment, the water may be contained in an amount of 40 wt% to 80 wt%. In a preferred embodiment, the water may be contained in an amount of 45 wt% to 70 wt% with respect to a total weight of the carbon dioxide.

In an exemplary embodiment, the amine-based compound may be contained in an amount of 10 wt% to 50 wt% with respect to a total weight of the carbon dioxide absorbent. In an exemplary embodiment, the amine-based compound may be contained in an amount of 20 wt% to 40 wt% with respect to a total weight of the carbon dioxide absorbent. In a preferred embodiment, the amine-based compound may be contained in an amount of 28 wt% to 32 wt%, with respect to a total weight of the carbon dioxide absorbent.

In another general aspect, a carbon dioxide separation method includes bringing the carbon dioxide absorbent according to an exemplary embodiment into contact with a mixture containing carbon dioxide under a temperature condition of 20°C to 80°C. In an exemplary embodiment, the temperature condition may be 20°C to 60°C, 20°C to 50°C, 30°C to 60°C, 30°C to 50°C, or about 40°C.

In an exemplary embodiment, the carbon dioxide separation method may further include desorbing carbon dioxide attached to the carbon dioxide absorbent by performing a heat treatment on the carbon dioxide absorbent under a temperature condition of 70°C to 150°C for 30 minutes to 250 minutes.

In an exemplary embodiment, in the carbon dioxide separation method, the bringing of the carbon dioxide absorbent into contact with the mixture and the desorbing of the carbon dioxide may be sequentially repeated to continuously separate the carbon dioxide.

In another general aspect, the carbon dioxide absorbent as disclosed herein is used in a carbon dioxide separation method. In an exemplary embodiment, the carbon dioxide absorbent is used in the carbon dioxide separation method as disclosed herein. In the present invention, the carbon dioxide absorbent as defined herein can be used for inhibiting decomposition of the carbon dioxide absorbent in the desorbing of the carbon dioxide.

In another general aspect, a method of preparing thecarbon dioxide absorbent as defined herein is disclosed.

Other features and aspects will be apparent from the following detailed description, the drawings, and the claims.

### DETAILED DESCRIPTION OF EMBODIMENTS

Exemplary embodiments described in the present specification may be modified in various different forms, and the technology according to one exemplary embodiment is not limited to the exemplary embodiments described below. Furthermore, throughout the specification, unless explicitly described to the contrary, "comprising", "including", "containing", or "having" any components will be understood to imply further inclusion of other components rather than the exclusion of any other components, and does not exclude elements, materials, or processes which are not further listed.

A numerical range used in the present specification includes upper and lower limits and all values within these limits, increments logically derived from a form and span of a defined range, all double limited values, and all possible combinations of the upper and lower limits in the numerical range defined in different forms. As an example, when a content of a composition is limited to 10% to 80% or 20% to 50%, a numerical range of 10% to 50% or 50% to 80% should also be interpreted as described in the present specification. Unless otherwise specifically defined in the present specification, values out of the numerical range that may occur due to experimental errors or rounded values also fall within the defined numerical range.

Hereinafter, in the present specification, unless otherwise specifically defined, "about" may be considered a value within 30%, 25%, 20%, 15%, 10%, 5%, 3%, 2%, 1%, or 0.5% of a stated value.

The term "alkylene group" as used in the present specification refers to a linear or branched diradical of a carbon saturated bond, and may be substituted with an arbitrary substituent.

The term "alkyl group" as used in the present specification refers to a linear or branched radical of a carbon saturated bond, and may be substituted with an arbitrary substituent.

The term "cycloalkyl group" as used in the present specification refers to a carbon ring radical of a carbon saturated bond, and may be substituted with an arbitrary substituent.

The term "heterocycloalkyl group" as used in the present specification refers to a ring radical containing one or more heteroatoms selected from the group consisting of oxygen (O), nitrogen (N), and sulfur (S), and may be substituted with an arbitrary substituent. For example, the "5-membered to 20-membered heterocycloalkyl group" means that the number of carbon, oxygen, nitrogen, and/or sulfur atoms forming the ring is 5 to 20 excluding the number of substituent atoms such as hydrogen atoms and similar atoms substituted on carbon atoms.

Hereinafter, the present disclosure will be described in detail (with reference to the accompanying drawings). However, the description is merely exemplary and the present disclosure is not limited to specific exemplary embodiments described by way of example.

The present invention provides a carbon dioxide absorbent containing an ionic material containing a cyclic ammonium cation represented by the following Chemical Formula 1 and a hydroxide anion:

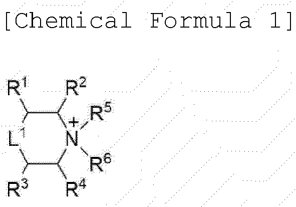

wherein
R¹ to R⁴ are each independently -H, a C₁₋₂₀ alkyl group, a C₁₋₂₀ alkoxy group, a C₁₋₁₀ alkoxy C₁₋₁₀ alkyl group, a C₅₋₂₀ cycloalkyl group, or a 5-membered to 20-membered heterocycloalkyl group; R⁵ and R⁶ are each independently -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group; L¹ is a single bond, -O-, -NR⁷-, or a C₁₋₈ alkylene group; R⁷ is -H or a C₁₋₁₀ alkyl group or R⁷ may be bonded to R⁵ to form a ring; and the C₁₋₂₀ alkyl group, the C₁₋₂₀ alkoxy group, the C₁₋₁₀ alkoxy C₁₋₁₀ alkyl group, the C₅₋₂₀ cycloalkyl group, and the 5-membered to 20-membered heterocycloalkyl group of R¹ to R⁷ may each independently be substituted with a halogen group, -OH, -NH₂, or -NO₂.

In an exemplary embodiment, the carbon dioxide absorbent comprises an ionic material as described herein, or the carbon dioxide absorbent consists of an ionic material that consists of a cyclic ammonium cation as described herein, and a hydroxide anion, and optionally further a solvent and an amine-based compound as described herein. The ionic material is obtained by combining an ammonium cation with a hydroxide anion (⁻OH), which has a comparably high basicity and a significantly small molecular weight compared to a non-hydroxide anion (i.e. an anion other than ⁻OH, such as an acetate-based anion, a phosphate-based anion, a halogen anion, a fluorine-based anion (BF₄⁻, PF₆⁻, or similar fluorine-based anions), NO₃⁻, or similar anions) that is used in a carbon dioxide absorbent of the prior art.

The carbon dioxide absorbent according to an exemplary embodiment contains an ionic material obtained by combining an ammonium cation with a hydroxide anion (OH⁻) having a comparably high basicity and a significantly small molecular weight compared to an anion (an acetate-based anion, a phosphate-based anion, a halogen anion, a fluorine-based anion (BF₄⁻, PF₆⁻, or the like), NO₃⁻, or similar fluorine-based anions) used in a carbon dioxide absorbent of the related art, such that the carbon dioxide capture (absorption) capacity per unit volume of the absorbent may be effectively increased.

In addition, since all the ionic materials contained in the carbon dioxide absorbent according to an exemplary embodiment are soluble in water and, thus, dissolve in a solvent before and after carbon dioxide absorption, layer separation may not occur. Meanwhile, an anion used in the related art, such as a phenolate anion, is converted to phenol by receiving a proton during a process in which the carbon dioxide absorbent absorbs carbon dioxide, and phenol does not dissolve in water, which causes the layer separation of the absorbent. The layer separation may cause instability of a flow rate of the absorbent within a continuous circulation apparatus, and an additional process such as adding an additional solvent is required to eliminate the layer separation, which is not economically effective compared to the carbon dioxide absorbent according to an exemplary embodiment.

In addition, the absorbent according to an exemplary embodiment significantly solves the problem of an increase in viscosity after absorption compared to before carbon dioxide absorption, thereby enabling carbon dioxide absorption and desorption to be performed significantly efficiently. In a case where phenol, carbamate, or carbonate is produced using the carbon dioxide absorbent of the related art, as a viscosity of a reaction solution increases, the efficiency of the continuous carbon dioxide absorption reaction is reduced, and high temperature conditions are required for a long period of time when desorbing carbon dioxide again after carbon dioxide absorption. However, the carbon dioxide absorbent according to an exemplary embodiment may be effective in carbon dioxide absorption and desorption since the viscosity does not significantly increase after capture.

In an exemplary embodiment, R¹ to R⁴ may each independently be -H, a C₁₋₁₅ alkyl group, a C₁₋₁₀ alkyl group, a C₁₋₈ alkyl group, a C₁₋₅ alkyl group, a C₁₋₃ alkyl group, a methyl group, an ethyl group, a C₁₋₁₅ alkoxy group, a C₁₋₁₀ alkoxy group, a C₁₋₈ alkoxy group, a C₁₋₅ alkoxy group, a C₁₋₃ alkoxy group, a methoxy group, an ethoxy group, a C₁₋₈ alkoxy C₁₋₈ alkyl group, a C₁₋₅ alkoxy C₁₋₅ alkyl group, a C₁₋₃ alkoxy C₁₋₃ alkyl group, an ethoxymethyl group, a methoxymethyl group, a C₅₋₁₅ cycloalkyl group, a C₅₋₁₀ cycloalkyl group, a C₅₋₈ cycloalkyl group, a C₆₋₈ cycloalkyl group, a cyclopentyl group, a cyclohexyl group, or a 5-membered to 15-membered, 5-membered to 10-membered, 5-membered to 8-membered, or 6-membered to 8-membered heterocycloalkyl group.

In an exemplary embodiment, R⁵ and R⁶ may each independently be -H, a C₁₋₁₅ alkyl group, a C₁₋₁₀ alkyl group, a C₁₋₈ alkyl group, a C₁₋₅ alkyl group, a C₁₋₃ alkyl group, a methyl group, an ethyl group, a C₁₋₈ alkoxy C₁₋₈ alkyl group, a C₁₋₅ alkoxy C₁₋₅ alkyl group, a C₁₋₃ alkoxy C₁₋₃ alkyl group, an ethoxymethyl group, a methoxymethyl group, a C₅₋₁₅ cycloalkyl group, a C₅₋₁₀ cycloalkyl group, a C₅₋₈ cycloalkyl group, a C₆₋₈ cycloalkyl group, a cyclopentyl group, a cyclohexyl group, or a 5-membered to 15-membered, 5-membered to 10-membered, 5-membered to 8-membered, or 6-membered to 8-membered heterocycloalkyl group.

In an exemplary embodiment, L¹ may be a single bond, -O-,-NR⁷-, a C₁₋₈ alkylene group, a C₁₋₃ alkylene group, a methylene group, or an ethylene group, and R⁷ may be -H, a C₁₋₁₀ alkyl group, a C₁₋₈ alkyl group, a C₁₋₅ alkyl group, a C₁₋₃ alkyl group, a methyl group, or an ethyl group, or R⁷ may be bonded to R⁵ to form a ring. In this case, when R⁷ is bonded to R⁵ to form a bicyclo ring, the cyclic ammonium cation may be represented by the following Chemical Formula 3:

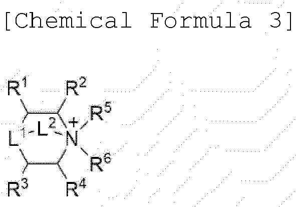

wherein the substituents R¹ to R⁶ and L¹ are as defined in Chemical Formula 1 or Chemical Formula 2, and L² may be a C₁₋₅ alkylene group, a C₁₋₃ alkylene group, a C₁₋₂ alkylene group, a methylene group, or an ethylene group.

In an exemplary embodiment, the alkyl group, the alkoxy group, the alkoxyalkyl group, the cycloalkyl group, and the heterocycloalkyl group of R¹ to R⁷ may each independently be substituted with an arbitrary substituent, and may be substituted with, for example, one or more halogen groups selected from the group consisting of I, Br, Cl, and F, -OH, -NH₂, or -NO₂.

In addition, in an exemplary embodiment, R¹ to R⁴ may each independently be -H, a C₁₋₁₀ alkyl group, a C₁₋₁₀ alkoxy group, a C₁₋₅ alkoxy C₁₋₅ alkyl group, a C₅₋₁₀ cycloalkyl group, or a 5-membered to 10-membered heterocycloalkyl group; R⁵ and R⁶ may each independently be -H, a C₁₋₁₀ alkyl group, a C₁₋₅ alkoxy C₁₋₅ alkyl group, a C₅₋₁₀ cycloalkyl group, or a 5-membered to 10-membered heterocycloalkyl group; L¹ may be a single bond, -O-,-NR⁷-, or a C₁₋₅ alkylene group; R⁷ may be -H or a C₁₋₅ alkyl group, or R⁷ may be bonded to R⁵ to form a ring; and the C₁₋₁₀ alkyl group, the C₁₋₁₀ alkoxy group, the C₁₋₅ alkoxy C₁₋₅ alkyl group, the C₅₋₁₀ cycloalkyl group, and the 5-membered to 10-membered heterocycloalkyl group of R¹ to R⁷ may each independently be substituted with a halogen group, -OH, -NH₂, or -NO₂.

In an exemplary embodiment, the cyclic ammonium cation may be represented by the following Chemical Formula 2:

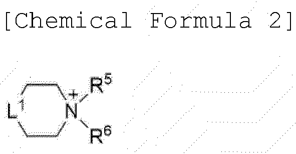

wherein
R⁵ and R⁶ may each independently be -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group; L¹ is a single bond, -O-, -NR⁷-, or a C₁₋₈ alkylene group; R⁷ is -H or a C₁₋₁₀ alkyl group, or R⁷ may be bonded to R⁵ to form a ring; and the C₁₋₁₀ alkyl group, the C₁₋₂₀ alkyl group, the C₁₋₁₀ alkoxy C₁₋₁₀ alkyl group, the C₅₋₂₀ cycloalkyl group, and the 5-membered to 20-membered heterocycloalkyl group of R⁵ to R⁷ may each independently be substituted with a halogen group, -OH, -NH₂, or -NO₂.

In an exemplary embodiment, the substituents R⁵, R⁶, and L¹ in Chemical Formula 2 may be as defined in Chemical Formula 1, and specifically, in an exemplary embodiment, R⁵ and R⁶ may each independently be a C₁₋₁₀ alkyl group, a C₁₋₅ alkoxy C₁₋₅ alkyl group, a C₅₋₁₀ cycloalkyl group, or a 5-membered to 10-membered heterocycloalkyl group; L¹ may be a single bond, -O-, -NR⁷-, or a C₁₋₈ alkylene group; R⁷ may be -H or a C₁₋₅ alkyl group; and the C₁₋₁₀ alkyl group, the C₁₋₅ alkoxy C₁₋₅ alkyl group, the C₅₋₁₀ cycloalkyl group, and the 5-membered to 10-membered heterocycloalkyl group of R⁵ to R⁷ may each independently be substituted with -OH, -NH₂, or -NO₂.

Alternatively, in an exemplary embodiment, in Chemical Formula 2, R⁵ and R⁶ are each independently a C₁₋₅ alkyl group or a C₁₋₃ alkoxy C₁₋₃ alkyl group; L¹ is -O- or a methylene group; and the C₁₋₅ alkyl group and the C₁₋₃ alkoxy C₁₋₃ alkyl group of R⁵ and R⁶ may each independently be substituted with -OH.

In an exemplary embodiment, the cyclic ammonium cation may be 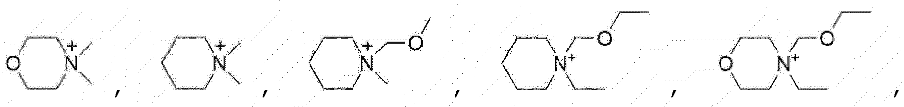 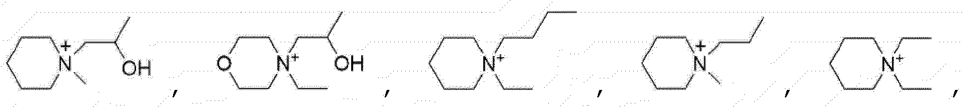 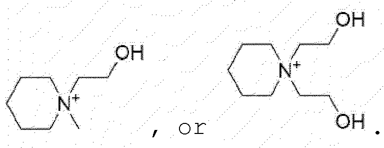 In a further exemplary embodiment, the cyclic ammonium cation may be

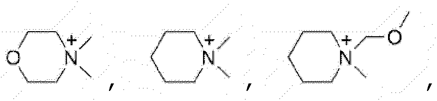

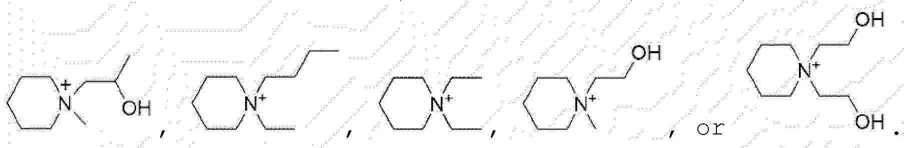

In an exemplary embodiment, the carbon dioxide absorbent may further contain water as a solvent. Alternatively, in an exemplary embodiment, the carbon dioxide absorbent may further contain one or more solvents other than water together with water as a solvent, for example, an amine-based compound. In an exemplary embodiment, the amine-based compound may include, but is not limited to, monoethanolamine (MEA), N-methyldiethanolamine (MDEA), diethanolamine (DEA), triethanolamine (TEA), 2-amino-2-methyl-1-propanol (AMP), and/or piperazine (PZ). In a preferred embodiment, the amine-based compound is piperazine. In an exemplary embodiment, the carbon dioxide absorbent may contain water as a solvent together with piperazine. Alternatively, the carbon dioxide absorbent may further contain a non-aqueous solvent, for example, ethylene glycol, propylene glycol, methyl glycol, methyl isopropyl carboxylate, methyl diethyl carboxylate, triethylene glycol, dimethyl sulfonate, and/or diethyl sulfonate.

In an exemplary embodiment, the ionic material may be contained in an amount of 5 wt% to 50 wt%, 5 wt% to 40 wt%, 10 wt% to 50 wt%, 10 wt% to 40 wt%, 20 wt% to 50 wt%, 20 wt% to 40 wt%, 25 wt% to 40 wt%, 25 wt% to 30 wt%, about 25 wt%, or about 30 wt%, with respect to a total weight of the carbon dioxide absorbent, but is not limited to the above range.

In an exemplary embodiment, in a case where the carbon dioxide absorbent contains water as a solvent, the water may be contained in an amount of 30 wt% to 90 wt% or 40 wt% to 80 wt% with respect to the total weight of the carbon dioxide absorbent, but is not limited to the above range. In an exemplary embodiment, in a case where the carbon dioxide absorbent contains only water as a solvent, the water may be contained in an amount of 50 wt% to 90 wt%, 50 wt% to 80 wt%, 60 wt% to 80 wt%, or about 70 wt%, but is not limited to the above range. In an exemplary embodiment, in a case where the carbon dioxide absorbent further contains a solvent other than water as a solvent, the water may be contained in an amount of 30 wt% to 60 wt%, 30 wt% to 50 wt%, 40 wt% to 50 wt%, or about 45 wt%, but is not limited to the above range.

In an exemplary embodiment, in a case where the carbon dioxide absorbent further contains a solvent other than water, the solvent other than water may be contained in an amount of 10 wt% to 50 wt%, 10 wt% to 40 wt%, 20 wt% to 50 wt%, 20 wt% to 40 wt%, 25 wt% to 40 wt%, 25 wt% to 30 wt%, about 25 wt%, or about 30 wt%, with respect to the total weight of the carbon dioxide absorbent, but is not limited to the above range, and the solvent other than water may be, for example, an amine-based compound.

In an exemplary embodiment, a capture (or absorption) performance (unit: mmol/mL) of the carbon dioxide absorbent may be 0.5 or more, 0.9 or more, 1.0 or more, 1.5 or more, 1.8 or more, 3.0 or more, 3.5 or more, 4.0 or more, or 4.3 or more. In this case, an upper limit thereof may be 2.0 or less, 3.0 or less, 4.8 or less, 5.0 or less, or 5.5 or less. The capture performance refers to the number of moles of carbon dioxide captured per unit volume of the carbon dioxide absorbent.

In an exemplary embodiment, a capture (or absorption) equivalent (unit: mol/mol) of the carbon dioxide absorbent may be 0.7 or more, 1.0 or more, 1.3 or more, 1.8 or more, or 2.0 or more. In this case, an upper limit thereof may be 4.0 or less, 3.0 or less, or 2.5 or less. The capture equivalent refers to the number of moles of carbon dioxide captured per unit mole of the ionic material and the amine-based compound in the carbon dioxide absorbent.

Another exemplary embodiment provides a carbon dioxide supply agent containing a compound formed by reacting carbon dioxide with the carbon dioxide absorbent.

Still another exemplary embodiment provides a carbon dioxide separation method including bringing the carbon dioxide absorbent according to an exemplary embodiment into contact with a mixture containing carbon dioxide under a temperature condition of 20°C to 80°C.

In an exemplary embodiment, the temperature condition may be, for example, 20°C to 60°C, 20°C to 50°C, 30°C to 60°C, 30°C to 50°C, or about 40°C. In addition, the bringing of the carbon dioxide absorbent into contact with the mixture may be performed under a pressure condition of 0.1 bar to 2.0 bar, 0.5 bar to 2.0 bar, 0.5 bar to 1.5 bar, 0.7 bar to 1.3 bar, or 1.0 bar, but is not limited thereto.

The carbon dioxide separation method may further include desorbing carbon dioxide attached to the absorbent by performing a heat treatment on the carbon dioxide absorbent under conditions of a temperature of 70°C to 150°C and a N₂ flow of 100 cc/min to 300 cc/min for 30 minutes to 250 minutes. In this case, the temperature condition is not limited to the above range, and for example, the heat treatment may be performed at 80°C to 130°C, 80°C to 120°C, 80°C to 110°C, 85°C to 120°C, 85°C to 110°C, or 90°C to 110°C. In addition, the time condition is not limited to the above range, and for example, the heat treatment may be performed for 30 minutes to 210 minutes, 50 minutes to 210 minutes, 60 minutes to 250 minutes, 60 minutes to 200 minutes, or 60 minutes to 180 minutes. In addition, the N₂ flow condition is not limited to the above range, and for example, the heat treatment may be performed at 150 cc/min to 250 cc/min, 180 cc/min to 220 cc/min, or 200 cc/min.

In addition, the carbon dioxide separation method may be a method of continuously separating the carbon dioxide by sequentially repeating the contact with the carbon dioxide to absorb the carbon dioxide and the desorbing of the carbon dioxide attached to the absorbent.

The carbon dioxide separation method according to an exemplary embodiment may reduce a reduction rate of the absorbent after carbon dioxide desorption due to inhibition of decomposition of the carbon dioxide absorbent in the desorbing of the carbon dioxide under high temperature and basic conditions. Accordingly, in the carbon dioxide separation method, the loss of the absorbent according to the number of processes is less than that of the carbon dioxide absorption method using the carbon dioxide absorbent of the related art, such that the amount of absorbent that needs to be replenished after each process may be reduced, and the process operation may be more economically effective.

Hereinafter, examples and experimental examples will be described in detail. However, the examples and the experimental examples described below are only illustrative of some exemplary embodiments, and the technology described in the present specification is not construed as being limited thereto.

### <Preparation Examples 1 and 2> Preparation of Ionic Materials

133.6 mmol of an iodide of each cation in Table 1, 133.6 mmol of silver(I) oxide, and 80 mL of water were added, and then, stirring was performed at room temperature for 4 hours. The produced silver iodide solid was removed by filtration, and an excess of water was dried under vacuum at 50°C, thereby obtaining an ionic material in an aqueous solution state. The ¹H NMR of the obtained ionic material was measured. The results thereof are shown in Table 1.

### <Preparation Example 3> Preparation of Ionic Material

133.6 mmol of a chloride of the cation in Table 1, 140.3 mmol of potassium hydroxide, and 80 mL of ethanol were added, and then, stirring was performed at room temperature for 4 hours. The produced potassium chloride solid was removed by filtration, an excess of water was added, and then, ethanol was removed by vacuum drying at 50°C, thereby obtaining an ionic material in an aqueous solution state. The ¹H NMR of the obtained ionic material was measured. The results thereof are shown in Table 1.

### <Preparation Example 4> Preparation of Ionic Material

133.6 mmol of N-methyl piperidine, 140.3 mmol of propylene oxide, and 80 mL of ethanol were added, and then, stirring was performed at room temperature for 4 hours. An excess of water was added, and then, ethanol was removed by vacuum drying at 50°C, thereby obtaining an ionic material in an aqueous solution state. The ¹H NMR of the obtained ionic material was measured. The results thereof are shown in Table 1.

### <Preparation Example 5> Preparation of Ionic Material

133.6 mmol of a bromide of the cation in Table 1, 133.6 mmol of silver(I) oxide, and 80 mL of water were added, and then, stirring was performed at room temperature for 4 hours. The produced silver bromide solid was removed by filtration, and an excess of water was dried under vacuum at 50°C, thereby obtaining an ionic material in an aqueous solution state. The ¹H NMR of the obtained ionic material was measured. The results thereof are shown in Table 1.

### <Preparation Example 6> Preparation of Ionic Material

133.6 mmol of ethyl sulfate salt of the cation in Table 1 and 6.68 mmol of sulfuric acid, and 80 mL of water were added, and then, refluxing was performed while performing stirring at 110°C for 16 hours. An excess of ethanol was added, water was removed by vacuum drying at 50°C, 140.3 mmol of potassium hydroxide and 80 mL of ethanol were added, and then, stirring was performed at room temperature for 4 hours. The produced potassium sulfate solid was removed by filtration, an excess of water was added, and then, ethanol was removed by vacuum drying at 50°C, thereby obtaining an ionic material in an aqueous solution state. The ¹H NMR of the obtained ionic material was measured. The results thereof are shown in Table 1.

### <Preparation Examples 7 and 8> Preparation of Ionic Materials

133.6 mmol of a chloride of each cation in Table 1, 140.3 mmol of potassium hydroxide, and 80 mL of ethanol were added, and then, stirring was performed at room temperature for 4 hours. The produced potassium chloride solid was removed by filtration, an excess of water was added, and then, ethanol was removed by vacuum drying at 50°C, thereby obtaining an ionic material in an aqueous solution state. The ¹H NMR of the obtained ionic material was measured. The results thereof are shown in Table 1.

**[Table 1]**

| Preparation Example No. | Ionic material | | ¹H NMR |
|---|---|---|---|
| | Cation | Anion | |
| 1 | 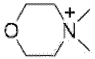 | ⁻OH | ¹H NMR (500 MHz), D₂O, 25°C: δ=4.10(t, 4H), 3.52(t, 4H), 3.15(s, 6H) |
| 2 | 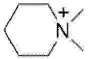 | ⁻OH | ¹H NMR (500 MHz), D₂O, 25°C: δ=3.25(t, 4H), 3.03(s, 6H), 1.80(br, 4H), 1.55(m, 2H) |
| 3 | 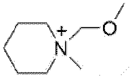 | ⁻OH | ¹H NMR (500 MHz), D₂O, 25°C: δ=4.63(s, 2H), 3.69(s, 3H), 3.25~3.38(m, 4H), 3.01(s, 3H), 1.90(br, 4H), 1.57~1.70(m, 2H) |
| 4 | 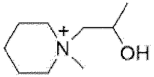 | ⁻OH | ¹H NMR (500 MHz), D₂O, 25°C: δ=4.33 (quintet, 1H), 3.43~3.22(m, 6H), 3.10~3.04 (m, 3H), 1.88~1.72(m, 4H), 1.59~1.56(m, 2H), 1.16(d, 3H) |
| 5 | 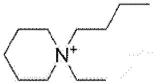 | ⁻OH | ¹H NMR (500 MHz), D₂O, 25°C: δ=3.33~3.45(m, 8H), 1.87(br, 4H), 1.66~1.73(m, 4H), 1.39~1.45(m, 2H), 1.27(t, 3H), 0.98(t, 3H) |
| 6 | 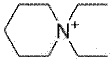 | ⁻OH | ¹H NMR (500 MHz), D₂O, 25°C: δ=3.32~3.41(m, 8H), 1.85(br, 4H), 1.67~1.75(m, 2H), 1.29(t, 6H) |
| 7 | 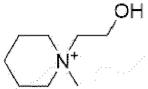 | ⁻OH | ¹H NMR (500 MHz), D₂O, 25°C: δ=4.06(s, 2H), 3.53(t, 2H), 3.48~3.38(m, 4H), 3.14(s, 3H), 1.91~1.89(m, 4H), 1.69~1.65(m, 2H) |
| 8 | 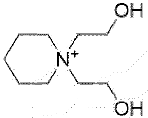 | ⁻OH | ¹H NMR (500 MHz), D₂O, 25°C: δ=4.05(t, 4H), 3.65(t, 4H), 3.52(t, 4H), 1.93~1.91(m, 4H), 1.72~1.67 (m, 2H) |

### <Preparation Example 9> Preparation of Ionic Material

133.6 mmol of N-methyl piperidine, 133.6 mmol of methyl iodide, and 40 mL of tetrahydrofuran were added, and then, stirring was performed at room temperature for 18 hours. Tetrahydrofuran was removed by vacuum drying at 50°C for 15 hours, and an ionic material in a solid state was obtained. The ¹H NMR of the obtained ionic material was measured. The results thereof are shown in Table 2.

### <Preparation Examples 10 and 11> Preparation of Ionic Materials

133.6 mmol of the ionic material of Preparation Example 2 in Table 1, 133.6 mmol of phenol or imidazole, and 40 mL of water were added, and then, stirring was performed at room temperature for 1 hour, thereby obtaining an ionic material in an aqueous solution state. The ¹H NMR of the obtained ionic material was measured. The results thereof are shown in Table 2.

### <Preparation Examples 12 to 13> Preparation of Ionic Materials

133.6 mmol of the ionic material of Preparation Example 9 in Table 2, 133.6 mmol of silver(I) acetate or silver(I) tetrafluoroborate, and 40 mL of water were added, and then, stirring was performed at room temperature for 4 hours. The produced silver iodide solid was removed by filtration and an ionic material in an aqueous solution state was obtained. The ¹H NMR of the obtained ionic material was measured. The results thereof are shown in Table 2.

### <Preparation Example 14> Preparation of Ionic Material

133.6 mmol of the ionic material of Preparation Example 2 in Table 1, 133.6 mmol of phosphoric acid, and 40 mL of water were added, and then, stirring was performed at room temperature for 1 hour, thereby obtaining an ionic material in an aqueous solution state. The ¹H NMR of the obtained ionic material was measured. The results thereof are shown in Table 2.

**[Table 2]**

| Preparation Example No. | Ionic material | | ¹H NMR |
|---|---|---|---|
| | Cation | Anion | |
| 9 | 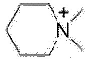 | I⁻ | ¹H NMR (500 MHz), D₂O, 25°C: δ=3.37(t, 4H), 3.20(s, 6H), 1.93(br, 4H), 1.70~81(m, 2H) |
| 10 | 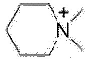 | 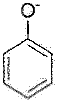 | ¹H NMR (500 MHz), D₂O, 25°C: δ=6.96(t, 2H), 6.43(d, 2H), 6.40(d, 1H), 3.20(t, 4H), 3.06(s, 6H), 1.83(br, 4H), 1.60(quintet, 2H) |
| 11 | 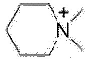 | 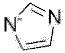 | ¹H NMR (500 MHz), D₂O, 25°C: δ=7.70(s, 1H), 7.07(s, 2H), 3.27 (t, 4H), 3.03(s, 6H), 1.82 (br, 4H), 1.57~61(m, 2H) |
| 12 | 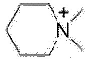 | 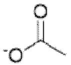 | ¹H NMR (500 MHz), D₂O, 25°C: δ=3.34(t, 4H), 3.10(s, 6H), 1.91(s, 3H), 1.88(br, 4H), 1.65(quintet, 2H) |
| 13 | 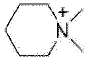 | BF₄⁻ | ¹H NMR (500 MHz), D₂O, 25°C: δ=3.38 (t, 4H), 3.14(s, 6H), 1.93(br, 4H), 1.70(quintet, 2H) |
| 14 | 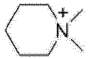 | 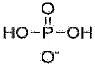 | ¹H NMR (500 MHz), D₂O, 25°C: δ=3.18 (t, 4H), 2.94 (s, 6H), 1.72 (br, 4H), 1.49 (quintet, 2H) |

### <Examples 1 to 16> Preparation of Carbon Dioxide Absorbents

Carbon dioxide absorbents of Examples 1 to 16 containing the ionic materials and solvents of Preparation Examples 1 to 8 with the compositions shown in Table 3 were prepared. Specifically, the carbon dioxide absorbent was prepared by adding the ionic material and the solvent at wt% (based on the total weight of the carbon dioxide absorbent) shown in Table 3, regardless of the order of addition, and performing mixing at a temperature of about 30°C for about 40 minutes.

**[Table 3]**

| Example No. | Ionic material (wt%) | Solvent (wt%) |
|---|---|---|
| 1 | Preparation Example 1 (30 wt%) | Water (70 wt%) |
| 2 | Preparation Example 2 (30 | Water (70 wt%) |
| | wt%) | |
| 3 | Preparation Example 3 (30 wt%) | Water (70 wt%) |
| 4 | Preparation Example 4 (30 wt%) | Water (70 wt%) |
| 5 | Preparation Example 5 (30 wt%) | Water (70 wt%) |
| 6 | Preparation Example 6 (30 wt%) | Water (70 wt%) |
| 7 | Preparation Example 7 (30 wt%) | Water (70 wt%) |
| 8 | Preparation Example 8 (30 wt%) | Water (70 wt%) |
| 9 | Preparation Example 1 (25 wt%) | Water (45 wt%), PZ (30 wt%) |
| 10 | Preparation Example 2 (25 wt%) | Water (45 wt%), PZ (30 wt%) |
| 11 | Preparation Example 3 (25 wt%) | Water (45 wt%), PZ (30 wt%) |
| 12 | Preparation Example 4 (25 wt%) | Water (45 wt%), PZ (30 wt%) |
| 13 | Preparation Example 5 (25 wt%) | Water (45 wt%), PZ (30 wt%) |
| 14 | Preparation Example 6 (25 wt%) | Water (45 wt%), PZ (30 wt%) |
| 15 | Preparation Example 7 (25 wt%) | Water (45 wt%), PZ (30 wt%) |
| 16 | Preparation Example 8 (25 wt%) | Water (45 wt%), PZ (30 wt%) |

| | | |
|---|---|---|
| (PZ: piperazine) | | |

### <Comparative Examples 1 to 12> Preparation of Carbon Dioxide Absorbents

Carbon dioxide absorbents of Comparative Examples 1 to 12 containing the ionic materials and solvents of Preparation Examples 9 to 14 with the compositions shown in Table 4 were prepared. Specifically, the carbon dioxide absorbent was prepared by adding the ionic material and the solvent at wt% (based on the total weight of the carbon dioxide absorbent) shown in Table 4, regardless of the order of addition, and performing mixing at a temperature of about 30°C for about 40 minutes.

**[Table 4]**

| Comparative Example No. | Ionic material (wt%) | Solvent (wt%) |
|---|---|---|
| 1 | Preparation Example 9 (30 wt%) | Water (70 wt%) |
| 2 | Preparation Example 10 (30 wt%) | Water (70 wt%) |
| 3 | Preparation Example 11 (30 wt%) | Water (70 wt%) |
| 4 | Preparation Example 12 (30 wt%) | Water (70 wt%) |
| 5 | Preparation Example 13 (30 wt%) | Water (70 wt%) |
| 6 | Preparation Example 14 (30 wt%) | Water (70 wt%) |
| 7 | Preparation Example 9 (25 wt%) | Water (45 wt%), PZ (30 wt%) |
| 8 | Preparation Example 10 (25 wt%) | Water (45 wt%), PZ (30 wt%) |
| 9 | Preparation Example 11 (25 wt%) | Water (45 wt%), PZ (30 wt%) |
| 10 | Preparation Example 12 (25 wt%) | Water (45 wt%), PZ (30 wt%) |
| 11 | Preparation Example 13 (25 wt%) | Water (45 wt%), PZ (30 wt%) |
| 12 | Preparation Example 14 (25 wt%) | Water (45 wt%), PZ (30 wt%) |

| | | |
|---|---|---|
| (PZ: piperazine) | | |

### <Experimental Example 1> Carbon Dioxide Absorption Performance Evaluation

In order to evaluate the carbon dioxide absorption performance of each of the carbon dioxide absorbents according to the examples and the comparative examples, the carbon dioxide absorption performance was measured using a vapor liquid equilibrium (VLE) apparatus. As the vapor liquid equilibrium apparatus, an apparatus including a carbon dioxide storage cylinder (150 mL), a constant temperature water bath, a stainless steel absorption reactor (73 mL) equipped with a thermometer, an electronic pressure gauge, and a stirrer was used. At this time, the absorption performance was measured while maintaining the cylinder and the reactor at a constant temperature of 40°C using a constant temperature water tank and a heating block, respectively. The measurement error ranges of the reactor were ±0.1°C and ±0.01 bar.

The carbon dioxide absorption performance evaluation was specifically performed using the following method. First, the inside of each of the carbon dioxide storage cylinder and the absorption reactor was sufficiently replaced with nitrogen, and then, the carbon dioxide storage cylinder was filled with carbon dioxide and maintained at 1 bar and 40°C. Next, a carbon dioxide absorbent (6.0 g) solution according to each of the examples and the comparative examples was injected into the absorption reactor, the temperature was maintained at 40°C, a valve connecting the cylinder and the reactor was opened, and then, the pressure was measured after absorption equilibrium was reached. The equilibrium pressure was measured every 30 minutes, and the process was repeated until there was no pressure change between the cylinder and the reactor. Next, the ideal gas equation was used to calculate the number of moles of carbon dioxide captured according to pressure change.

The values obtained by dividing the calculated number of moles (mmol) of carbon dioxide captured by the volume (mL) of the carbon dioxide absorbent injected are shown in Tables 5 and 6. In addition, after evaluating the carbon dioxide absorption performance, the presence or absence of layer separation in the absorbent was visually observed and the results were shown together.
X denotes that there is no layer separation present (absence of layer separation), and O denotes that there is a layer separation (presence of layer separation).

**[Table 5]**

| Example No. | Capture performance (mmol/mL) | Presence or absence of layer separation | Example No. | Capture performance (mmol/mL) | Presence or absence of layer separation |
|---|---|---|---|---|---|
| 1 | 2.16 | X | 9 | 4.64 | X |
| 2 | 2.32 | X | 10 | 4.68 | X |
| 3 | 1.83 | X | 11 | 4.45 | X |
| 4 | 1.60 | X | 12 | 4.21 | X |
| 5 | 1.61 | X | 13 | 4.33 | X |
| 6 | 2.03 | X | 14 | 4.36 | X |
| 7 | 1.79 | X | 15 | 4.42 | X |
| 8 | 1.63 | X | 16 | 4.29 | X |

**[Table 6]**

| Comparative Example No. | Capture performance (mmol/mL) | Presence or absence of layer separation | Comparative Example No. | Capture performance (mmol/mL) | Presence or absence of layer separation |
|---|---|---|---|---|---|
| 1 | 0.00 | X | 7 | 3.39 | X |
| 2 | 0.86 | O | 8 | 4.07 | X |
| 3 | 1.47 | X | 9 | 3.93 | X |
| 4 | 0.03 | X | 10 | 3.21 | X |
| 5 | 0.00 | X | 11 | 3.05 | O |
| 6 | 0.00 | X | 12 | 2.23 | Solid precipitation |

As can be confirmed through Tables 5 and 6, the carbon dioxide capture capacity values of Examples 1 to 8 which used only ionic materials containing a cyclic ammonium cation and a hydroxide anion (with the ionic material prepared as described in Preparation Examples 1 to 8) are all 1.6 mmol-CO₂/mL-absorbent or more, showing the improved carbon dioxide absorption performance compared to Comparative Examples 1 to 6 which used only ionic materials containing a cyclic ammonium cation and a non-hydroxide anion (i.e. using an anion other than hydroxide; with the ionic material prepared as described in Preparation Examples 9 to 14). In addition, it can be seen that the carbon dioxide capture capacity values of Examples 9 to 16, in which an amine-based compound was additionally used in the solvent, are all 4.2 mmol-CO₂/mL-absorbent or more, showing the improved carbon dioxide absorption performance compared to Comparative Examples 7 to 12. In addition, since no layer separation is observed in all of the absorbents of the examples, there is no factor that causes the instability of the flow rate of the absorbent within the continuous circulation apparatus, and there is no need for a process of adding an additional solvent to resolve the layer separation, which is much more economically effective.

An exemplary embodiment relates to a carbon dioxide absorbent containing an ionic material containing a cyclic ammonium cation and a hydroxide anion. The carbon dioxide absorbent according to an exemplary embodiment contains a hydroxide anion having a small molecular weight and high basicity, such that absorption performance per unit volume of the absorbent may be effectively improved. Further, the carbon dioxide absorbent according to an exemplary embodiment is soluble in water and thus may not cause layer separation problems.

Hereinabove, although an exemplary embodiment has been described in detail by the examples and the experimental examples, the scope of the exemplary embodiment is not limited to a specific example, and should be construed by the appended claims.

## Claims

1. A carbon dioxide absorbent comprising an ionic material containing a cyclic ammonium cation represented by the following Chemical Formula 1 and a hydroxide anion: 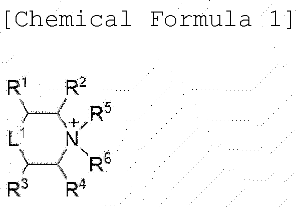 wherein
R¹ to R⁴ are each independently -H, a C₁₋₂₀ alkyl group, a C₁₋₂₀ alkoxy group, a C₁₋₁₀ alkoxy C₁₋₁₀ alkyl group, a C₅₋₂₀ cycloalkyl group, or a 5-membered to 20-membered heterocycloalkyl group;
R⁵ and R⁶ are each independently -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group;
L¹ is a single bond, -O-, -NR⁷-, or a C₁₋₈ alkylene group;
R⁷ is -H or a C₁₋₁₀ alkyl group, or R⁷ may be bonded to R⁵ to form a ring; and
the C₁₋₂₀ alkyl group, the C₁₋₂₀ alkoxy group, the C₁₋₁₀ alkoxy C₁₋₁₀ alkyl group, the C₅₋₂₀ cycloalkyl group, and the 5-membered to 20-membered heterocycloalkyl group of R¹ to R⁷ may optionally each independently be substituted with a halogen group, -OH, -NH₂, or -NO₂.

2. The carbon dioxide absorbent of claim 1, wherein in Chemical Formula 1,
R¹ to R⁴ are each independently -H, a C₁₋₁₀ alkyl group, a C₁₋₁₀ alkoxy group, a C₁₋₅ alkoxy C₁₋₅ alkyl group, a C₅₋₁₀ cycloalkyl group, or a 5-membered to 10-membered heterocycloalkyl group;
R⁵ and R⁶ are each independently -H, a C₁₋₁₀ alkyl group, a C₁₋₅ alkoxy C₁₋₅ alkyl group, a C₅₋₁₀ cycloalkyl group, or a 5-membered to 10-membered heterocycloalkyl group;
L¹ is a single bond, -O-, -NR⁷-, or a C₁₋₅ alkylene group;
R⁷ is -H or a C₁₋₅ alkyl group, or R⁷ may be bonded to R⁵ to form a ring; and
the C₁₋₁₀ alkyl group, the C₁₋₁₀ alkoxy group, the C₁₋₅ alkoxy C₁₋₅ alkyl group, the C₅₋₁₀ cycloalkyl group, and the 5-membered to 10-membered heterocycloalkyl group of R¹ to R⁷ may optionally each independently be substituted with a halogen group, -OH, -NH₂, or -NO₂.

3. The carbon dioxide absorbent of claim 1, wherein the cyclic ammonium cation is represented by the following Chemical Formula 2: 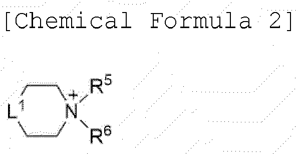 wherein
R⁵ and R⁶ are each independently -H, a C₁₋₂₀ alkyl group, a C₁₋₁₀ alkoxy C₁₋₁₀ alkyl group, a C₅₋₂₀ cycloalkyl group, or a 5-membered to 20-membered heterocycloalkyl group;
L¹ is a single bond, -O-, -NR⁷-, or a C₁₋₈ alkylene group;
R⁷ is -H or a C₁₋₁₀ alkyl group, or R⁷ may be bonded to R⁵ to form a ring; and
the C₁₋₁₀ alkyl group, the C₁₋₂₀ alkyl group, the C₁₋₁₀ alkoxy C₁₋₁₀ alkyl group, the C₅₋₂₀ cycloalkyl group, and the 5-membered to 20-membered heterocycloalkyl group of R⁵ to R⁷ may optionally each independently be substituted with a halogen group, -OH, -NH₂, or -NO₂.

4. The carbon dioxide absorbent of claim 3, wherein in Chemical Formula 2,
R⁵ and R⁶ are each independently a C₁₋₁₀ alkyl group, a C₁₋₅ alkoxy C₁₋₅ alkyl group, a C₅₋₁₀ cycloalkyl group, or a 5-membered to 10-membered heterocycloalkyl group;
L¹ is a single bond, -O-, -NR⁷-, or a C₁₋₈ alkylene group;
R⁷ is -H or a C₁₋₅ alkyl group; and
the C₁₋₁₀ alkyl group, the C₁₋₅ alkoxy C₁₋₅ alkyl group, the C₅₋₁₀ cycloalkyl group, and the 5-membered to 10-membered heterocycloalkyl group of R⁵ to R⁷ may optionally each independently be substituted with -OH, -NH₂, or -NO₂.

5. The carbon dioxide absorbent of claim 3, wherein in Chemical Formula 2,
R⁵ and R⁶ are each independently a C₁₋₅ alkyl group or a C₁₋₃ alkoxy C₁₋₃ alkyl group;
L¹ is -O- or a methylene group; and
the C₁₋₅ alkyl group and the C₁₋₃ alkoxy C₁₋₃ alkyl group of R⁵ and R⁶ may optionally each independently be substituted with - OH.

6. The carbon dioxide absorbent of claim 1, wherein the cyclic ammonium cation is any one selected from the group consisting of the following compounds: 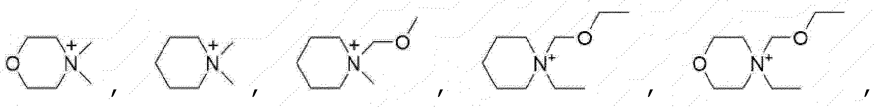 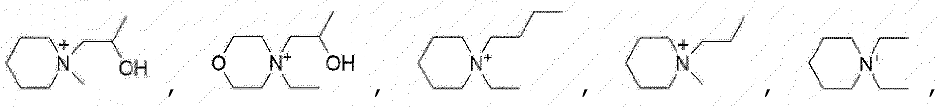 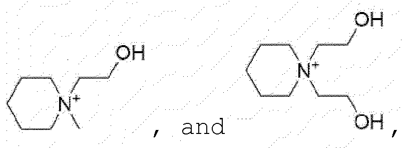 preferably, the cyclic ammonium cation is any one selected from the group consisting of the following compounds:

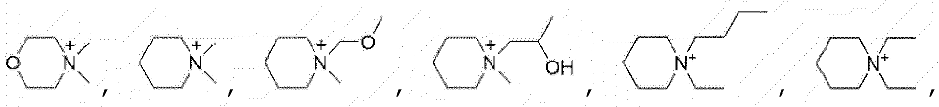

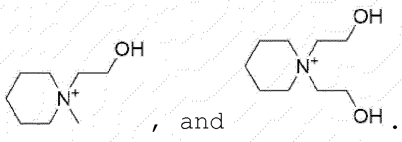

7. The carbon dioxide absorbent of any one of the preceding claims, further comprising water, and/or wherein the carbon dioxide absorbent further comprises one or more solvents other than the water.

8. The carbon dioxide absorbent of claim 7, wherein the solvent contains an amine-based compound; preferably wherein the amine-based compound includes one or more selected from the group consisting of monoethanolamine (MEA), N-methyldiethanolamine (MDEA), diethanolamine (DEA), triethanolamine (TEA), 2-amino-2-methyl-1-propanol (AMP), and piperazine (PZ), more preferably the amine-based compound includes piperazine.

9. The carbon dioxide absorbent of any one of the preceding claims, wherein the ionic material is contained in an amount of 5 wt% to 50 wt% with respect to a total weight of the carbon dioxide absorbent.

10. The carbon dioxide absorbent of claim 7, wherein water is included and the water is contained in an amount of 30 wt% to 90 wt% with respect to a total weight of the carbon dioxide absorbent.

11. The carbon dioxide absorbent of claim 8, wherein the amine-based compound is contained in an amount of 10 wt% to 50 wt% with respect to a total weight of the carbon dioxide absorbent.

12. A carbon dioxide separation method comprising a step of bringing the carbon dioxide absorbent of any one of claims 1 to 11 into contact with a mixture containing carbon dioxide under a temperature condition of 20°C to 80°C.

13. The carbon dioxide separation method of claim 12, further comprising a step of desorbing carbon dioxide attached or absorbed to the carbon dioxide absorbent by performing a heat treatment on the carbon dioxide absorbent under a temperature condition of 70°C to 150°C for 30 minutes to 250 minutes, optionally wherein the bringing of the carbon dioxide absorbent into contact with the mixture and the desorbing of the carbon dioxide are sequentially repeated to continuously separate the carbon dioxide.

14. Use of a carbon dioxide absorbent as defined in any one of claims 1 to 11 for inhibiting decomposition of the carbon dioxide absorbent in the desorbing of the carbon dioxide.

15. Method of preparing a carbon dioxide absorbent as defined in any one of claims 1 to 11 comprising a step of combining the ionic material containing a cyclic ammonium cation and a hydroxide anion as defined in any one of claims 1 to 6 with a solvent as defined in any one of claims 7 or 8, optionally further comprising a step of mixing the ionic material and the solvent under a temperature condition of 20°C to 80°C.

## Patentansprüche

1. Kohlendioxidabsorptionsmittel, umfassend ein ionisches Material, das ein durch die folgende Chemische Formel 1 dargestelltes zyklisches Ammoniumkation und ein Hydroxidanion enthält: 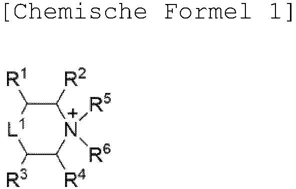 wobei
R¹ bis R⁴ jeweils unabhängig -H, eine C₁₋₂₀-Alkylgruppe, eine C₁₋₂₀₋Alkoxygruppe, eine C₁₋₁₀-Alkoxy-C₁₋₁₀-alkylgruppe, eine C₅₋₂₀₋Cycloalkylgruppe oder eine 5-gliedrige bis 20-gliedrige Heterocycloalkylgruppe sind;
R⁵ und R⁶ jeweils unabhängig -H, eine C₁₋₂₀₋Alkylgruppe, eine C₁₋₁₀-Alkoxy-C₁₋₁₀-alkylgruppe, eine C₅₋₂₀₋Cycloalkylgruppe oder eine 5-gliedrige bis 20-gliedrige Heterocycloalkylgruppe sind;
L¹ eine Einfachbindung, -O-, -NR⁷- oder eine C₁₋₈-Alkylengruppe ist;
R⁷ -H oder eine C₁₋₁₀-Alkylgruppe ist oder R⁷ an R⁵ gebunden sein kann, um einen Ring zu bilden; und
die C₁₋₂₀-Alkylgruppe, die C₁₋₂₀₋Alkoxygruppe, die C₁₋₁₀₋ Alkoxy-C₁₋₁₀-alkylgruppe, die C₅₋₂₀₋Cycloalkylgruppe und die 5-gliedrige bis 20-gliedrige Heterocycloalkylgruppe von R¹ bis R⁷ optional jeweils unabhängig mit einer Halogengruppe, -OH, -NH₂ oder -NO₂ substituiert sein können.

2. Kohlendioxidabsorptionsmittel gemäß Anspruch 1, wobei in der Chemischen Formel 1
R¹ bis R⁴ jeweils unabhängig -H, eine C₁₋₁₀-Alkylgruppe, eine C₁₋₁₀₋Alkoxygruppe, eine C₁₋₅₋Alkoxy-C₁₋₅-alkylgruppe, eine C₅₋₁₀₋Cycloalkylgruppe oder eine 5-gliedrige bis 10-gliedrige Heterocycloalkylgruppe sind;
R⁵ und R⁶ jeweils unabhängig -H, eine C₁₋₁₀-Alkylgruppe, eine C₁₋₅₋Alkoxy-C₁₋₅-alkylgruppe, eine C₅₋₁₀₋Cycloalkylgruppe oder eine 5-gliedrige bis 10-gliedrige Heterocycloalkylgruppe sind;
L¹ eine Einfachbindung, -O-, -NR⁷- oder eine C₁₋₅-Alkylengruppe ist;
R⁷ -H oder eine C₁₋₅-Alkylgruppe ist oder R⁷ an R⁵ gebunden sein kann, um einen Ring zu bilden; und
die C₁₋₁₀-Alkylgruppe, die C₁₋₁₀₋Alkoxygruppe, die C₁₋₅₋ Alkoxy-C₁₋₅-alkylgruppe, die C₅₋₁₀₋Cycloalkylgruppe und die 5-gliedrige bis 10-gliedrige Heterocycloalkylgruppe von R¹ bis R⁷ optional jeweils unabhängig mit einer Halogengruppe, -OH, -NH₂ oder -NO₂ substituiert sein können.

3. Kohlendioxidabsorptionsmittel gemäß Anspruch 1, wobei das zyklische Ammoniumkation durch die folgende Chemische Formel 2 dargestellt ist: 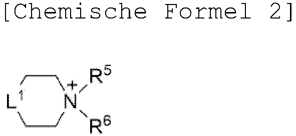 wobei
R⁵ und R⁶ jeweils unabhängig -H, eine C₁₋₂₀₋Alkylgruppe, eine C₁₋₁₀-Alkoxy-C₁₋₁₀-alkylgruppe, eine C₅₋₂₀₋Cycloalkylgruppe oder eine 5-gliedrige bis 20-gliedrige Heterocycloalkylgruppe sind;
L¹ eine Einfachbindung, -O-, -NR⁷- oder eine C₁₋₈-Alkylengruppe ist;
R⁷ -H oder eine C₁₋₁₀-Alkylgruppe ist oder R⁷ an R⁵ gebunden sein kann, um einen Ring zu bilden; und
die C₁₋₁₀-Alkylgruppe, die C₁₋₂₀-Alkylgruppe, die C₁₋₁₀₋ Alkoxy-C₁₋₁₀-alkylgruppe, die C₅₋₂₀₋Cycloalkylgruppe und die 5-gliedrige bis 20-gliedrige Heterocycloalkylgruppe von R⁵ bis R⁷ optional jeweils unabhängig mit einer Halogengruppe, -OH, -NH₂ oder -NO₂ substituiert sein können.

4. Kohlendioxidabsorptionsmittel gemäß Anspruch 3, wobei in der Chemischen Formel 2
R⁵ und R⁶ jeweils unabhängig eine C₁₋₁₀-Alkylgruppe, eine C₁₋₅₋Alkoxy-C₁₋₅-alkylgruppe, eine C₅₋₁₀₋Cycloalkylgruppe oder eine 5-gliedrige bis 10-gliedrige Heterocycloalkylgruppe sind;
L¹ eine Einfachbindung, -O-, -NR⁷- oder eine C₁₋₈-Alkylengruppe ist;
R⁷ -H oder eine C₁₋₅-Alkylgruppe ist; und
die C₁₋₁₀-Alkylgruppe, die C₁₋₅₋Alkoxy-C₁₋₅-alkylgruppe, die C₅₋₁₀₋Cycloalkylgruppe und die 5-gliedrige bis 10-gliedrige Heterocycloalkylgruppe von R⁵ bis R⁷ optional jeweils unabhängig mit -OH, -NH₂ oder -NO₂ substituiert sein können.

5. Kohlendioxidabsorptionsmittel gemäß Anspruch 3, wobei in der Chemischen Formel 2
R⁵ und R⁶ jeweils unabhängig eine C₁₋₅-Alkylgruppe oder eine C₁₋₃-Alkoxy-C₁₋₃-alkylgruppe sind;
L¹ -O- oder eine Methylengruppe ist; und
die C₁₋₅-Alkylgruppe und die C₁₋₃-Alkoxy-C₁₋₃-alkylgruppe von R⁵ und R⁶ optional jeweils unabhängig mit -OH substituiert sein können.

6. Kohlendioxidabsorptionsmittel gemäß Anspruch 1, wobei das zyklische Ammoniumkation eines ist, ausgewählt aus der Gruppe, bestehend aus den folgenden Verbindungen: 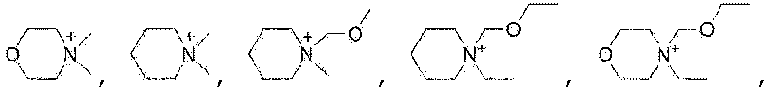 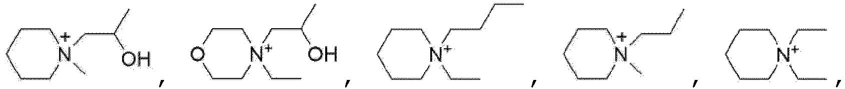 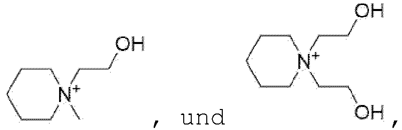 vorzugsweise ist das zyklische Ammoniumkation eines, ausgewählt aus der Gruppe, bestehend aus den folgenden Verbindungen:

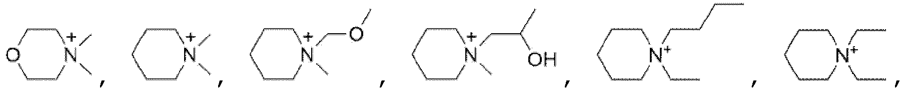

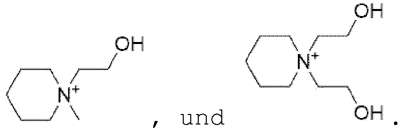

7. Kohlendioxidabsorptionsmittel gemäß einem der vorstehenden Ansprüche, ferner umfassend Wasser, und/oder wobei das Kohlendioxidabsorptionsmittel ferner ein oder mehrere andere Lösungsmittel als das Wasser umfasst.

8. Kohlendioxidabsorptionsmittel gemäß Anspruch 7, wobei das Lösungsmittel eine Verbindung auf Aminbasis enthält; vorzugsweise wobei die Verbindung auf Aminbasis eines oder mehrere ausgewählt aus der Gruppe bestehend aus Monoethanolamin (MEA), N-Methyldiethanolamin (MDEA), Diethanolamin (DEA), Triethanolamin (TEA), 2-Amino-2-methyl-1-propanol (AMP) und Piperazin (PZ) umfasst, wobei die Verbindung auf Aminbasis stärker bevorzugt Piperazin enthält.

9. Kohlendioxidabsorptionsmittel gemäß einem der vorstehenden Ansprüche, wobei das ionische Material in einer Menge von 5 Gew.-% bis 50 Gew.-%, bezogen auf das Gesamtgewicht des Kohlendioxidabsorptionsmittels, enthalten ist.

10. Kohlendioxidabsorptionsmittel gemäß Anspruch 7, wobei Wasser enthalten ist und das Wasser in einer Menge von 30 Gew.-% bis 90 Gew.-%, bezogen auf das Gesamtgewicht des Kohlendioxidabsorptionsmittels, enthalten ist.

11. Kohlendioxidabsorptionsmittel gemäß Anspruch 8, wobei die Verbindung auf Aminbasis in einer Menge von 10 Gew.-% bis 50 Gew.-%, bezogen auf das Gesamtgewicht des Kohlendioxidabsorptionsmittels, enthalten ist.

12. Kohlendioxidtrennverfahren, umfassend einen Schritt des Inkontaktbringens des Kohlendioxidabsorptionsmittels gemäß einem der Ansprüche 1 bis 11 mit einer Mischung, die Kohlendioxid enthält, unter einer Temperaturbedingung von 20 °C bis 80 °C.

13. Kohlendioxidtrennverfahren gemäß Anspruch 12, ferner umfassend einen Schritt des Desorbierens von Kohlendioxid, das mit dem Kohlendioxidabsorptionsmittel verbunden oder davon absorbiert ist, durch Durchführen einer Wärmebehandlung an dem Kohlendioxidabsorptionsmittel unter einer Temperaturbedingung von 70 °C bis 150 °C für 30 Minuten bis 250 Minuten, wobei optional das Inkontaktbringen des Kohlendioxidabsorptionsmittels mit der Mischung und das Desorbieren des Kohlendioxids nacheinander wiederholt werden, um das Kohlendioxid kontinuierlich zu trennen.

14. Verwendung eines Kohlendioxidabsorptionsmittels wie in einem der Ansprüche 1 bis 11 festgelegt zum Inhibieren der Zersetzung des Kohlendioxidabsorptionsmittels beim Desorbieren des Kohlendioxids.

15. Verfahren zur Herstellung eines Kohlendioxidabsorptionsmittels wie in einem der Ansprüche 1 bis 11 festgelegt, umfassend einen Schritt des Kombinierens des ionischen Materials, das ein zyklisches Ammoniumkation und ein Hydroxidanion enthält, wie in einem der Ansprüche 1 bis 6 festgelegt, mit einem Lösungsmittel wie in einem der Ansprüche 7 oder 8 festgelegt, optional ferner umfassend einen Schritt des Mischens des ionischen Materials und des Lösungsmittels unter einer Temperaturbedingung von 20 °C bis 80 °C.

## Revendications

1. Absorbant de dioxyde de carbone comprenant un matériau ionique contenant un cation ammonium cyclique représenté par la Formule Chimique 1 suivante et un anion hydroxyde : 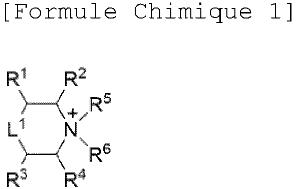 où
R¹ à R⁴ sont chacun indépendamment -H, un groupe alkyle en C₁₋₂₀, un groupe alcoxy en C₁₋₂₀, un groupe alcoxy en C₁₋₁₀-alkyle en C₁₋₁₀, un groupe cycloalkyle en C₅₋₂₀, ou un groupe hétérocycloalkyle de 5 chaînons à 20 chaînons ;
R⁵ et R⁶ sont chacun indépendamment -H, un groupe alkyle en C₁₋₂₀, un groupe alcoxy en C₁₋₁₀-alkyle en C₁₋₁₀, un groupe cycloalkyle en C₅₋₂₀, ou un groupe hétérocycloalkyle de 5 chaînons à 20 chaînons ;
L¹ est une liaison simple, -O-, -NR⁷-, ou un groupe alkylène en C₁₋₈ ;
R⁷ est -H ou un groupe alkyle en C₁₋₁₀, ou R⁷ peut être lié à R⁵ pour former un cycle ; et
le groupe alkyle en C₁₋₂₀, le groupe alcoxy en C₁₋₂₀, le groupe alcoxy en C₁₋₁₀-alkyle en C₁₋₁₀, le groupe cycloalkyle en C₅₋₂₀, et le groupe hétérocycloalkyle de 5 chaînons à 20 chaînons de R¹ à R⁷ peuvent éventuellement être chacun indépendamment substitués par un groupe halogène, -OH, -NH₂, ou -NO₂.

2. Absorbant de dioxyde de carbone selon la revendication 1, où dans la Formule Chimique 1,
R¹ à R⁴ sont chacun indépendamment -H, un groupe alkyle en C₁₋₁₀, un groupe alcoxy en C₁₋₁₀, un groupe alcoxy en C₁₋₅-alkyle en C₁₋₅, un groupe cycloalkyle en C₅₋₁₀, ou un groupe hétérocycloalkyle de 5 chaînons à 10 chaînons ;
R⁵ et R⁶ sont chacun indépendamment -H, un groupe alkyle en C₁₋₁₀, un groupe alcoxy en C₁₋₅-alkyle en C₁₋₅, un groupe cycloalkyle en C₅₋₁₀, ou un groupe hétérocycloalkyle de 5 chaînons à 10 chaînons ;
L¹ est une liaison simple, -O-, -NR⁷-, ou un groupe alkylène en C₁₋₅ ;
R⁷ est -H ou un groupe alkyle en C₁₋₅, ou R⁷ peut être lié à R⁵ pour former un cycle ; et
le groupe alkyle en C₁₋₁₀, le groupe alcoxy en C₁₋₁₀, le groupe alcoxy en C₁₋₅-alkyle en C₁₋₅, le groupe cycloalkyle en C₅₋₁₀, et le groupe hétérocycloalkyle de 5 chaînons à 10 chaînons de R¹ à R⁷ peuvent éventuellement être chacun indépendamment substitués par un groupe halogène, -OH, -NH₂, ou -NO₂.

3. Absorbant de dioxyde de carbone selon la revendication 1, où le cation ammonium cyclique est représenté par la Formule Chimique 2 suivante : 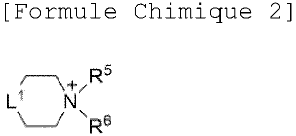 où
R⁵ et R⁶ sont chacun indépendamment -H, un groupe alkyle en C₁₋₂₀, un groupe alcoxy en C₁₋₁₀-alkyle en C₁₋₁₀, un groupe cycloalkyle en C₅₋₂₀, ou un groupe hétérocycloalkyle de 5 chaînons à 20 chaînons ;
L¹ est une liaison simple, -O-, -NR⁷-, ou un groupe alkylène en C₁₋₈ ;
R⁷ est -H ou un groupe alkyle en C₁₋₁₀, ou R⁷ peut être lié à R⁵ pour former un cycle ; et
le groupe alkyle en C₁₋₁₀, le groupe alkyle en C₁₋₂₀, le groupe alcoxy en C₁₋₁₀-alkyle en C₁₋₁₀, le groupe cycloalkyle en C₅₋₂₀, et le groupe hétérocycloalkyle de 5 chaînons à 20 chaînons de R⁵ à R⁷ peuvent éventuellement être chacun indépendamment substitués par un groupe halogène, -OH, -NH₂, ou -NO₂.

4. Absorbant de dioxyde de carbone selon la revendication 3, où dans la Formule Chimique 2,
R⁵ et R⁶ sont chacun indépendamment un groupe alkyle en C₁₋₁₀, un groupe alcoxy en C₁₋₅-alkyle en C₁₋₅, un groupe cycloalkyle en C₅₋₁₀, ou un groupe hétérocycloalkyle de 5 chaînons à 10 chaînons ;
L¹ est une liaison simple, -O-, -NR⁷-, ou un groupe alkylène en C₁₋₈ ;
R⁷ est -H ou un groupe alkyle en C₁₋₅ ; et
le groupe alkyle en C₁₋₁₀, le groupe alcoxy en C₁₋₅-alkyle en C₁₋₅, le groupe cycloalkyle en C₅₋₁₀, et le groupe hétérocycloalkyle de 5 chaînons à 10 chaînons de R⁵ à R⁷ peuvent éventuellement être chacun indépendamment substitués par -OH, -NH₂, ou -NO₂.

5. Absorbant de dioxyde de carbone selon la revendication 3, où dans la Formule Chimique 2,
R⁵ et R⁶ sont chacun indépendamment un groupe alkyle en C₁₋₅ ou un groupe alcoxy en C₁₋₃-alkyle en C₁₋₃ ;
L¹ est -O- ou un groupe méthylène ; et
le groupe alkyle en C₁₋₅ et le groupe alcoxy en C₁₋₃-alkyle en C₁₋₃ de R⁵ et R⁶ peuvent éventuellement être chacun indépendamment substitués par -OH.

6. Absorbant de dioxyde de carbone selon la revendication 1, où le cation ammonium cyclique est l'un quelconque sélectionné dans le groupe consistant en les composés suivants : 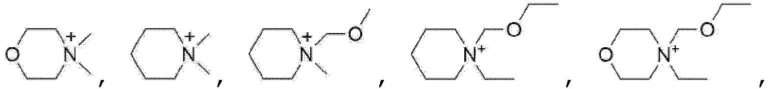 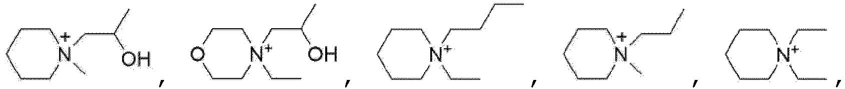 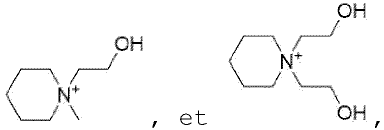 de préférence, le cation ammonium cyclique est l'un quelconque sélectionné dans le groupe consistant en les composés suivants :

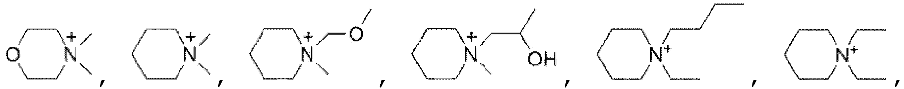

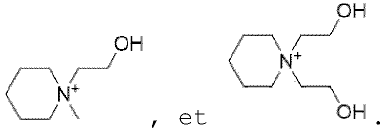

7. Absorbant de dioxyde de carbone selon l'une quelconque des revendications précédentes, comprenant en outre de l'eau, et/ou où l'absorbant de dioxyde de carbone comprend en outre un ou plusieurs solvants autres que l'eau.

8. Absorbant de dioxyde de carbone selon la revendication 7, où le solvant contient un composé à base d'amine ; de préférence où le composé à base d'amine inclut un ou plusieurs sélectionnés dans le groupe consistant en la monoéthanolamine (MEA), la N-méthyldiéthanolamine (MDEA), la diéthanolamine (DEA), la triéthanolamine (TEA), le 2-amino-2-méthyl-1-propanol (AMP), et la pipérazine (PZ), plus préférablement le composé à base d'amine inclut la pipérazine.

9. Absorbant de dioxyde de carbone selon l'une quelconque des revendications précédentes, où le matériau ionique est contenu en une quantité de 5 % en poids à 50 % en poids par rapport à un poids total de l'absorbant de dioxyde de carbone.

10. Absorbant de dioxyde de carbone selon la revendication 7, où de l'eau est incluse et l'eau est contenue en une quantité de 30 % en poids à 90 % en poids par rapport à un poids total de l'absorbant de dioxyde de carbone.

11. Absorbant de dioxyde de carbone selon la revendication 8, où le composé à base d'amine est contenu en une quantité de 10 % en poids à 50 % en poids par rapport à un poids total de l'absorbant de dioxyde de carbone.

12. Procédé de séparation de dioxyde de carbone comprenant une étape de mise en contact de l'absorbant de dioxyde de carbone selon l'une quelconque des revendications 1 à 11 avec un mélange contenant du dioxyde de carbone dans des conditions de température de 20 °C à 80 °C.

13. Procédé de séparation de dioxyde de carbone selon la revendication 12, comprenant en outre une étape de désorption de dioxyde de carbone attaché ou absorbé à l'absorbant de dioxyde de carbone en effectuant un traitement thermique sur l'absorbant de dioxyde de carbone dans des conditions de température de 70 °C à 150 °C pendant 30 minutes à 250 minutes, éventuellement où la mise en contact de l'absorbant de dioxyde de carbone avec le mélange et la désorption du dioxyde de carbone sont répétées séquentiellement pour séparer en continu le dioxyde de carbone.

14. Utilisation d'un absorbant de dioxyde de carbone tel que défini dans l'une quelconque des revendications 1 à 11 pour inhiber la décomposition de l'absorbant de dioxyde de carbone dans la désorption du dioxyde de carbone.

15. Procédé de préparation d'un absorbant de dioxyde de carbone tel que défini dans l'une quelconque des revendications 1 à 11 comprenant une étape de combinaison du matériau ionique contenant un cation ammonium cyclique et un anion hydroxyde tel que défini dans l'une quelconque des revendications 1 à 6 avec un solvant tel que défini dans l'une quelconque des revendications 7 ou 8, éventuellement comprenant en outre une étape de mélange du matériau ionique et du solvant dans des conditions de température de 20 °C à 80 °C.
